# EUROPEAN PATENT APPLICATION

(11) **EP 4 328 561 A1**
(43) Date of publication of application: **28.02.2024**
(21) Application number: 22791171.6
(22) Date of filing: 31.03.2022
(51) Int. Cl.: G01N 1/22, G01N 29/036, G01N 33/569, B01D 47/02, C12Q 1/24, G01G 3/13

(54) **BIOSENSOR AND VIRUSOMETER FOR THE DETECTION OF PATHOGENS IN AIR AND LIQUID SAMPLES**

(30) Priority: 22.04.2021 ES 202130357
(71) Applicant: Universitat Politècnica de València, 46022 Valencia (ES); Universitat de València, 46010 Valencia (ES)
(72) Inventor: MAQUIEIRA CATALA, Ángel, 46022 Valencia (ES); GIMENEZ ROMERO, David, 46010 Valencia (ES); JUSTE DOLZ, Augusto Miguel, 46022 Valencia (ES); MORAIS EZQUERRO, Sergi Beñat, 46022 Valencia (ES); SENA TORRALBA, Amadeo, 46022 Valencia (ES); TORTAJADA GENARO, Luis Antonio, 46022 Valencia (ES); BAÑULS POLO, María José, 46022 Valencia (ES); LUCIO BENITO, María Isabel, 46022 Valencia (ES); FERNÁNDEZ SÁNCHEZ, María Estrella, 46022 Valencia (ES); PALLÁS TAMARIT, Yeray, 46022 Valencia (ES)
(74) Representative: Pons
(86) International application number: PCT/ES2022/070191
(87) International publication number: WO 2022/223860

(57) **Abstract**

The invention relates to a biosensor and virusometer that allow continuous, real-time detection of viral particles of pathogens, especially SARS-CoV-2, both in indoor and outdoor air and in liquid samples in which the pathogen(s) have been collected. The biosensor (8) comprises a quartz crystal microbalance coated with a layer having one or more antibodies specific to one or more pathogens to be detected. A first virusometer (1) is described, comprising an aerosol collector (5), which makes it possible to increase the concentration of pathogens before taking them to the biosensor (8). A second virusometer comprises a receptacle (14), divided into a first cavity (15) wherein air exhaled by a user is collected and concentrated, and a second cavity (16) wherein the biosensor (8) is arranged.

## Description

### OBJECT OF THE INVENTION

This invention relates to the development of a biosensor and virusometers to carry out direct, continuous and real-time detection without marking, of viral particles of pathogens, especially SARS-CoV-2 (or other viruses and airborne pathogens, such as fungi and bacteria), which may be present in the air, forming aerosols, or in a liquid medium. The measurement can be direct, in air samples, or indirect, in liquid samples, wherein the virus present in the air is previously captured.

### BACKGROUND OF THE INVENTION

The transmission mechanisms of SARS-CoV-2 and other viruses and pathogens (fungi and bacteria) include transmission through droplets exhaled directly by patients, contact with contaminated surfaces, and through the inhalation of aerosols, especially in indoor environments.

This last transmission mechanism has been shown to be the main transmission route of SARS-CoV-2, by aerosols in indoor environments. Evidence from laboratory studies has shown airborne transmission of the COVID-19 virus being infectious in the air for prolonged periods of time.

The virus is detected by PCR *(Polymerase Chain Reaction*) in the air and in an aquatic medium. Researchers have reported values for the SARS-CoV-2 viral load in the mouth ranging from 10² to 10¹¹ copies per ml of respiratory fluid. Loads vary throughout the course of the disease, tending to reach a maximum close to the onset of symptoms.

Currently, there is no study that demonstrates transmission by droplets, however, there are several studies that demonstrate transmission by aerosols. The best-known case is that of the Skagit choir (USA), in which fifty-two people out of the sixty who participated in a rehearsal got infected. Consequently, preventive control strategies must consider aerosol transmission for effective mitigation of SARS-CoV-2.

In the midst of a race to achieve widespread vaccination of the world population against this coronavirus, it is possible we are protected from symptoms, but not from infection. It is possible for a vaccinated person to behave like an asymptomatic patient and transmit SARS-CoV-2.

Therefore, it is necessary to continue case detection, active contact tracing and preventive control, as this is the most effective way to control the COVID-19 pandemic, as well as future pandemics.

Detection methods should be:
- Sensitive to mild and symptomatic infections, in order to promote effective isolation and reduce transmission within risk groups;
- Consistent, to reliably monitor the presence of the virus and assist in clinical decision making; and
- Scalable, to provide information about local and national public health policies, such as when social distancing measures can be safely relaxed.

However, current SARS-CoV-2 detection strategies often fail to meet these criteria, in part because they rely on nasopharyngeal and oropharyngeal swabs as the most commonly used sample type to detect the virus using RT-PCR *(Reverse Transcription Polymerase Chain Reaction).*

Although throat swabs are commonly used in COVID-19 diagnostic sampling, it is an uncomfortable practice for patients due to the invasiveness of the procedure, which limits compliance with repetitive testing, making sampling difficult and posing considerable risks to health-care workers.

RT-PCR does not directly determine the presence of SARS-CoV-2 virions, which makes it difficult to estimate the load of SARS-CoV-2 in atmospheric aerosols, making this technique an ineffective warning method.

With regard to analyses of other viruses in air, for example, influenza A and B, there are quantitative culture methods to know the infectious viral load in samples collected from air. This method, although used as a reference, is not the most suitable for environmental monitoring and has multiple drawbacks, since it requires several days for cultivation and is laborious.

A series of known documents from the state of the art related to the stated problem, and the patent proposal under examination, are described below.

The document by Do Nascimento NM et al., (Label-free piezoelectric biosensor for prognosis and diagnosis of Systemic Lupus Erythematosus*),* describes the development of a biosensor useful for the diagnosis and prediction of systemic lupus erythematosus. The biosensor consists of a quartz crystal microbalance with dissipation monitoring of anti-TRIM21 or anti-TROVE2 circulating antibodies. The autoantigen capable of binding to the previous antibodies is immobilised by covalent anchoring, forming a self-assembled monolayer.

This is a work that is done entirely in solution, like most of the works done on a quartz crystal microbalance.

The Pandey LM document (Design of engineered surfaces for prospective detection of SARS-CoV-2 using quartz crystal microbalance-based techniques*)* discloses a device based on a quartz crystal microbalance for the detection of SARS-CoV-2 in oral swab samples. The authors base their document on previous knowledge of the structure of the viral spike protein and suggest the use of a self-assembled monolayer with COOH groups, to promote a strong and specific binding with the S1 region of said spike protein.

This work mentions different strategies for the detection of SARS-CoV-2 in solution. Among them, it specifies the detection of influenza A and B in clinical samples and cultured preparations, but always in solution.

The article by Narita F et al. (A Review of Piezoelectric and Magnetostrictive Biosensor Materials for Detection of COVID-19 and Other Viruses*)* contains a review of the application of piezoelectric immunosensors, which comprises quartz crystal microbalances (with or without dissipation) for the detection of different types of viruses, and suggests the use of this technology for developing portable devices useful for the detection of SARS- CoV-2. In this regard, it is necessary to point out that the article, after revision, does not provide any evidence that what is suggested works and can be operative, based on either the frequency signal or the dissipation factor. Furthermore these authors do not specify resonators to be used, reagents, measurement conditions and material means to achieve the result, since the published information is collected and expressed as conclusions, but not experimentally demonstrated, and from the publications cited by these authors (Narita et al., 2021), the same is not demonstrated for direct measurement of exhaled or aspirated air.

As in previous cases, it is key to know that reference is made to the detection of viruses in solution. In another case like the statement in Anal. Chem, Vol 76, No. 13, July 1, 2004, Boli Zuo, et al., detection in sputum, which is previously dissolved in water and then aerosolised, is discussed. It is, therefore, a very different approach to directly measuring breath. In addition, they use the frequency signal for their tests. This work presents surprising data since: It does not analyse or discuss viruses - It does the following: "Antigen powder, dried by lyophilization at-70 °C, was dissolved in the sputum of a healthy person" In other words, it does not work with an SARS virus, or anything similar. In turn, it mentions that "the frequency shifts were linearly dependent on antigen concentration in the range of 0.6-4 µg/ml." Although the limit of quantification is lower, the operating working range indicated by the authors corresponds to a concentration of the order of mg/l of antigen. It is unrealistic and impossible to have this level of virus in air or the antigen selected by the authors. The results are very relevant" Table 3. Responses of Ten Sensors to Reference Sputum and Antigen Sputum (1 µg/µl) at 25 °C." In other words, at 1g/l. The only thing this publication shows is what is already known and that is that the piezoelectric sensor detects the antibody-antigen interaction.

Owen TW et al. (Microgravimetric immunosensor for direct detection of aerosolized influenza A virus particles*)* disclose a piezoelectric immunosensor for detection of influenza A virus (H₃N₂) in aerosols. To capture the virus on the surface of the transducer, specific polyclonal antibodies against the virus are used. To construct the sensor surface, they form a self-assembled monolayer on the gold electrode of a quartz crystal microbalance and use EDC/NHS for covalent anchoring of the antibody. In this article, the quartz crystal microbalance is used for the direct detection of aerosolised influenza A virions. To do so, aqueous samples of the influenza virus are aerosolised using a nebuliser. This fluid is taken to the QCM measurement cell. The system does not allow for discrimination between viruses present in the atmosphere, and only detects high loads of aerosolised virions when using the sensor in frequency mode.

Verreault D et al. (Methods for sampling of airborne viruses*)* and McDermott H.J. (Air monitoring for toxic exposures*)* describe commercial sampling devices for capturing airborne viral particles in liquid. These devices operate by introducing high speed air into a container in which the particles are captured in an aqueous liquid.

### DESCRIPTION OF THE INVENTION

Viruses and other pathogenic microorganisms must be controlled in the atmosphere, especially in order to take measures against natural infections and even control possible bioterrorist attacks. From this perspective, a biosensor and virusometers for rapid detection of pathogens in air and in air exhaled by a patient, such as SARS-CoV-2, which have high sensitivity and selectivity, are described below as objects of the present invention.

Thus, a first object of the present invention is a biosensor for the direct detection of airborne pathogens, which comprises a quartz crystal microbalance or piezoelectric balance, which in turn comprises a layer with one or more specific antibodies for the pathogen to be detected. The specific antibodies are preferably covalently anchored on the surface of the microbalance or, alternatively, immobilised by another generic antibody, specific anti-antibody or a protein, hapten or bioreceptor, capable of immobilising the specific antibody, either randomly or in a directed and oriented manner. For example, protein A or similar, lectins, etc. They will preferably be specific anti-SARS-CoV-2 antibodies.

Microbalances of this type measure mass through changes in the frequency of a piezoelectric quartz crystal when it is disturbed by the change in mass due to the product that is intended to be measured. In addition, quartz crystal microbalances are also capable of providing information on the viscoelastic properties of the deposited material, which are determined by the dissipation factor recorded.

The biosensor of the invention is used especially for measuring the virus in a preferably closed environment. It records the number of virions present in the occupied spaces and provides proof of their presence in the air.

The biosensor allows for a triple verification, since it can be linked to a processing module, which allows for the measurement of three parameters: frequency shift (Δf), variation in the dissipation factor (ΔD) and the Δf/ΔD coefficient, and in this way, when the airborne virion interacts with antibodies, it causes a change in the resonance frequency and dissipation factor of the microbalance. Said change, for a single virus, must provide a constant Δf/ΔD coefficient over time, and specific to the interacting pathogen.

In this way, the pathogen of interest, particularly SARS-CoV-2, can be discerned and quantified from possible interferences. A detection of the SARS-CoV-2 virus involves a simultaneous change in resonance frequency and dissipation factor. To increase the sensitivity of the biosensor, the absolute frequency and dissipation values are not directly determined, but rather the rate of change of both parameters are determined.

In turn, the second object of the invention is a virusometer for the detection of pathogens that are also in the air, which makes use of the biosensor described, yet with the difference in that now a sample treatment and virus concentration step is envisaged. Thus, active sampling equipment is incorporated to capture microorganisms preferably in an absorbent liquid medium.

Specifically, the virusometer for the detection of pathogens in air comprises a sampling module, and a sample collection module and a virus detection module, linked to each other and associated with the sample collection module.

The sampling module comprises a first duct, with an air inlet and an air outlet, a flow measurement unit and fluid mobilisation means, preferably a vacuum pump, both arranged in the first duct.

In turn, the sample collection module, connected to the first duct of the sampling module, comprises an aerosol collector; meaning a gas bubbler or scrubber that selectively retains viruses and other pathogens and airborne materials in a preferably liquid medium, generally aqueous.

Lastly, the virus detection module comprises a second duct, connected to the aerosol collector in which fluid propulsion means are arranged, preferably a peristaltic pump, and an acoustic transducer, which comprises a biosensor, such as the one described in the first object of the invention.

The operation of the virusometer for the detection of pathogens in air is such that air enters through the air inlet of the sampling module. The aerosol collector and the vacuum pump are subsequently arranged to force the air stream to pass through the collection module. Lastly, the flow measurement unit is located in the first duct to measure the linear volumetric flow of the air that passes through the collection module.

The first duct must be as short as possible, without elbows and restricted parts where particulate or liquid material could accumulate.

The pump or vacuum line is used to capture the pathogens of interest in a collection liquid, preferably located in the aerosol collector. Next, the collected pathogens are transported through the second duct, thanks to the peristaltic pump, to the acoustic transducer, which monitors the flow at controlled periods of time.

The acoustic transducer comprises a biosensor, as described in the first object of the present invention, which comprises a quartz crystal balance, with a layer of a specific antibody derived from or related to the pathogen, the presence of which is to be detected in air.

In this way, when the pathogen interacts with the antibodies, it causes a change in the resonance frequency and dissipation factor of the acoustic transducer. The acoustic transducer of the invention can be associated with a data processing module, and is triple-verified.

Said change, for a single pathogen, must provide a constant Δf/ΔD coefficient over time, specific to the interacting virus. This ratio thus provides a control system for the acquired signal, as it allows the SARS-CoV-2 virus to be discerned and quantified from possible interferences.

The previous concentration in the liquid of the aerosol collector for pathogens present in air allows for a greater detection sensitivity, the virusometer for the detection of pathogens in air being suitable for measurements even outdoors.

The airborne pathogen detection virusometer operates in a collection fluid recirculation environment, thus making it useful for determining the concentration of the SARS-CoV-2 virion. The frequencies measurement is done with great precision, in a simple way, and thus it is easy to measure low concentrations.

Consequently, the SARS-CoV-2 virus contained in the atmosphere can be detected in real time, preferably in closed environments, such as hospitals, classrooms, business premises and even means of transportation, allowing appropriate measures to be taken.

Lastly, a third object of the present invention is a virusometer for the detection of pathogens in air exhaled by a user, preferably to detect SARS-CoV-2 in exhaled air, operating in real time, trapping the virus directly in the piezoelectric quartz biosensor, described in the first object of the invention, also coated with a specific antibody. The antibody is preferably immobilised by covalent anchoring, as described above. See page 5, paragraph 5 and page 11, paragraph 5 for details of this procedure.

The virusometer for the detection of pathogens in air exhaled by a user detects a pathogen, preferably SARS-CoV-2, by measuring the viral load directly in the exhaled air. It records the number of virions present in the air exhaled by the patient and provides a real-time measurement of the presence of pathogens.

To do this, there is a mouthpiece through which the user blows once or twice and a receptacle, divided by a movable separation piece into a first cavity and a second cavity. The mouthpiece is connected to the first cavity, so that air fills this first cavity when the user exhales. This facilitates the homogenisation and preconcentration of the pathogen before the measurement. Furthermore, this receptacle makes it so no cross contamination occurs between samples and the measurements are more homogeneous and reproducible.

Thus, when the blowing is finished, the separation element moves so that the air fills the second cavity, in which the biosensor for the detection of pathogens in air is arranged, as described in the first object of the invention.

The biosensor, which can be connected to processing means to store and send data, identifies the pathogen load contained in the exhaled air, taking the measurement at an interval of three minutes per test.

As in the previously described biosensor and virusometer for the detection of pathogens in air, when the airborne pathogen or virion interacts with the antibodies, it causes a change in the resonance frequency and dissipation factor of the biosensor.

As indicated, the virusometer for the detection of pathogens in air exhaled by a user allows for the detection of other airborne viruses and microorganisms (fungi and bacteria), mainly in aerosols, using antibodies or other bioreceptors that are specific for the desired pathogen. Furthermore, by immobilising different specific bioreceptors, several pathogens can be simultaneously determined.

In the present invention, "pathogen" means any virus or microorganism, prokaryotic or eukaryotic, including, but not limited to, bacteria, yeast and fungi, as well as parts thereof (for example, proteins, spores, virus-like particles, etc.), which are airborne, preferably in aerosols, and which are capable of infecting humans and/or animals, causing a disease. Examples of pathogens that can be detected by the objects of the present invention are, but are not limited to, those that cause measles, Covid-19, MRSA, influenza, avian flu, chickenpox or tuberculosis. Examples of these pathogens are *Varicella zoster, Mycobacterium tuberculosis, Candida spp., Aspergillus spp., Influenza virus,* SARS-CoV-2, etc.

The term "antibody" refers to both immunoglobulins and immunologically active portions thereof, for example, molecules containing an antigen binding site that specifically binds (immunoreacts) with the antigen. Examples of these portions include F(ab) and F(ab')2 fragments, which can be generated by treating the antibody with an enzyme, such as pepsin or recombinantly. The antibodies referred to in the present invention may also be recombinant, chimeric or synthetic. Optionally, the antibody to which the invention relates may be conjugated with a detectable marker.

In short, both the virusometer for the detection of pathogens in air and the virusometer for the detection of pathogens in air exhaled by the user are based on the previous concentration of the pathogens, which are then brought into contact with the biosensor. Therefore, the three objects of the present invention solve the existing problems in the direct and continuous detection of the SARS-CoV-2 virus, either from the antigens or other components thereof, in indoor and outdoor environments, without resorting to PCR analysis.

These types of measurements are essential in an environment like the current pandemic. Ventilation and specifically the renewal of indoor air are essential aspects related to the health and comfort of homes and work or leisure spaces. With the use of the proposed biosensors, unhealthy areas or rooms can be identified, based on the excessive levels of SARS-CoV-2.

Furthermore, virusometers are very versatile and allow several microorganisms to be detected simultaneously; for example, airborne viruses such as measles, influenza, avian flu and other pathologies caused by bacteria and fungi, such as tuberculosis and candidiasis, respectively. In all cases, detection is carried out through the use of specific or selective receptors for the pathogen or pathogens.

### DESCRIPTION OF THE DRAWINGS

As a complement to the description provided and for the purpose of helping to make the features of the invention more readily understandable, in accordance with a practical preferred exemplary embodiment thereof, said description is accompanied by a set of drawings which, by way of illustration and not limitation, represent the following:
Figure 1 shows a schematic representation of the virusometer for the detection of pathogens in air (SARS-CoV-2) in a liquid medium from air.
Figure 2 shows the temporal evolution of the frequency and dissipation signals of the acoustic transducer after eight minutes of sampling in a chamber in which PBS has been nebulised 1x. It corresponds to the recording obtained from a clean atmosphere.
Figure 3 shows the temporal evolution of the frequency and dissipation signals of the acoustic transducer after eight minutes of sampling in a chamber in which PBS 1x + [VPLs] = 10⁸ pfu/ml has been nebulised. It corresponds to the recording obtained from an atmosphere with viruses (VLPs).
Figure 4 shows a general view of the components of the virusometer used for the direct detection of pathogens in aerosols with a respiratory origin.
Figure 5 shows the temporal recording of the signal of the breath dissipation D factor of a COVID-19 patient with PCR + and a control subject (PCR -).
Figure 6 shows the temporal recording of the signal of the breath dissipation D factor of a COVID-19 patient with PCR + and a patient with pneumonia (CRP -).

### PREFERRED EMBODIMENT OF THE INVENTION

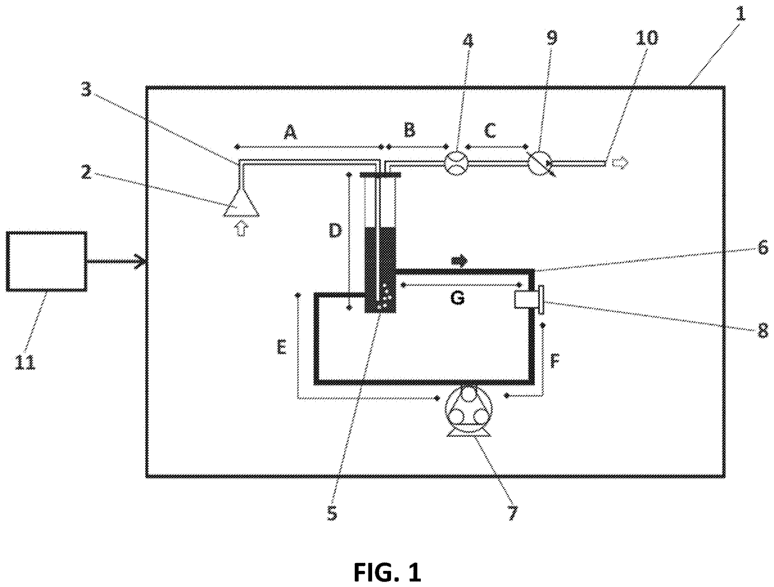

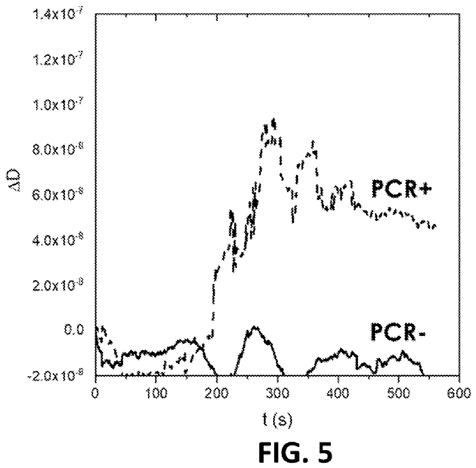

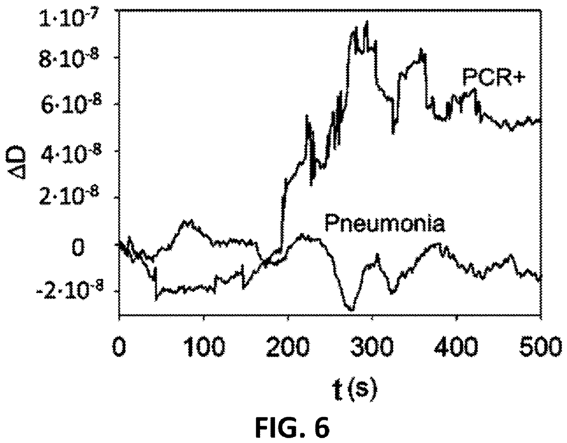

A preferred embodiment of the biosensor (8), the virusometer (1) for the detection of pathogens in air and the virusometer for the detection of SARS CoV-2 in air exhaled by a patient is described below, with the help of Figures 1 to 6., objects of the present invention.

A first object of the present invention is a biosensor (8) for the direct detection of virions in the air, which comprises a quartz crystal microbalance or piezoelectric balance, which in turn, comprises a layer with one or more anti-SARS-CoV-2 antibodies.

The biosensor (8) allows for a triple verification. In this way, when the airborne virion interacts with antibodies, it causes a change in the resonance frequency (f) and in the dissipation factor (D)) of the microbalance. Said change, for the same virus, provides a constant *Δf*/*ΔD* coefficient over time and specific to the interacting pathogen.

A detection of the SARS-CoV-2 virus involves a simultaneous change in resonance frequency and dissipation factor. The Δf/ΔD value must correspond approximately to - 12.8 MHz, a value optimised by using SARS-CoV-2 virus-like particles (VLPs).

The specific antibody is immobilised on the microbalance by covalent anchoring, forming a self-assembled monolayer. To form the monolayer and anchor the bioreceptor, antibodies in this case, different strategies widely found in the literature can be used. In this specific case, the monolayer was created by immersing the piezoelectric surface for 16 h in a 10 mM mercaptopropionic acid solution and the subsequent activation by 10 mM EDC/NHS for 60 min.

The surface was then treated with 5 mM of carbohydrazide for 60 min. Subsequently, 100 µl of the specific antibody (33 mg/I) was dispersed on the surface of the microbalance chip, being incubated for 60 min.

In turn, a second object of the present invention is a virusometer (1) for the detection of pathogens in air which, as shown in Figure 1, comprises a sampling module, and a sample collection module and a virus detection module, linked together and associated with the sample collection module. Furthermore, the virusometer (1) for the detection of pathogens in air is intended to be connected to an external device (11) for remote control and data collection.

The sampling module comprises a first duct (3), with an air inlet (2) and an air outlet (10), a flow measurement unit (4), a flow meter, and first fluid mobilisation means (7), such as a vacuum pump or connection to a general vacuum line, both arranged in the first duct (3).

The air inlet (2) includes a funnel or similar piece that prevents coarse particles from entering the first duct (3). The first duct (3) is a single-layer plasticised PVC tube, with a diameter of ten millimetres.

In turn, the sample collection module, connected to the first duct (3) of the sampling module, comprises an aerosol collector (5) that contains a collection liquid. The aerosol collector (5) that receives the air collected through the inlet (2) of the first duct (3) has a height of *D*∼18cm. However, this height must be adjusted if the airflow is changed.

Lastly, the virus detection module comprises a second duct (6), connected to the bubbler (5), and in which second fluid mobilisation means (7) are arranged, such as a peristaltic pump, and an acoustic transducer. The flow rate of the peristaltic pump is approximately 50 µl/min.

The second duct (6) is divided into a first section (E) that goes from the aerosol collector (5) to the peristaltic pump and is approximately 20 cm, a second section (F) from the peristaltic pump to the acoustic transducer, which is approximately 15 cm, and a third section (G) between the transducer and the aerosol collector (5), of about 20 cm.

In turn, the first duct (3) is also divided into three sections. A first section between the inlet (2) and the aerosol collector (5) of approximately 2 m, a second section between the aerosol collector (5) and the flow meter of approximately 40 cm, and a third section between the flow meter and the vacuum pump of approximately 40 cm.

As shown in Figure 1, the operation of the virusometer (1) for the detection of pathogens in air is such that air enters through the air inlet (2) of the sampling module. The aerosol collector (5) and the vacuum pump are subsequently arranged to force the air stream to pass through the collection module. Lastly, the flow measurement unit (4) is located in the first duct (3) to measure the linear volumetric flow of the gas that passes through the collection module.

The vacuum pump (9), positioned in the first duct (3), after the flow measurement unit (4) is used to capture the virus in the collection liquid located in the aerosol collector (5). Next, the collected sample is transported through the second duct (6) by the peristaltic pump, to the acoustic transducer, which monitors the flow at controlled periods of time.

The acoustic transducer comprises a biosensor (8) as the first object of the invention, which comprises a layer with one or several specific anti-SARS-CoV-2 antibodies. The acoustic transducer of the invention is triple verified. In this way, when the self-transported virion interacts with the antibodies, it causes a change in the resonance frequency and dissipation factor of the biosensor (8). Said change, for a single virus, must provide a constant Δf/ΔD coefficient over time, and specific to the interacting virus.

A detection of the SARS-CoV-2 virus involves a simultaneous change in resonance frequency and dissipation factor. Furthermore, the Δf/ΔD value must correspond to -5 ·10⁶, a value optimised by using VLPs *(Virus-like Particles)* of SARS-CoV-2.

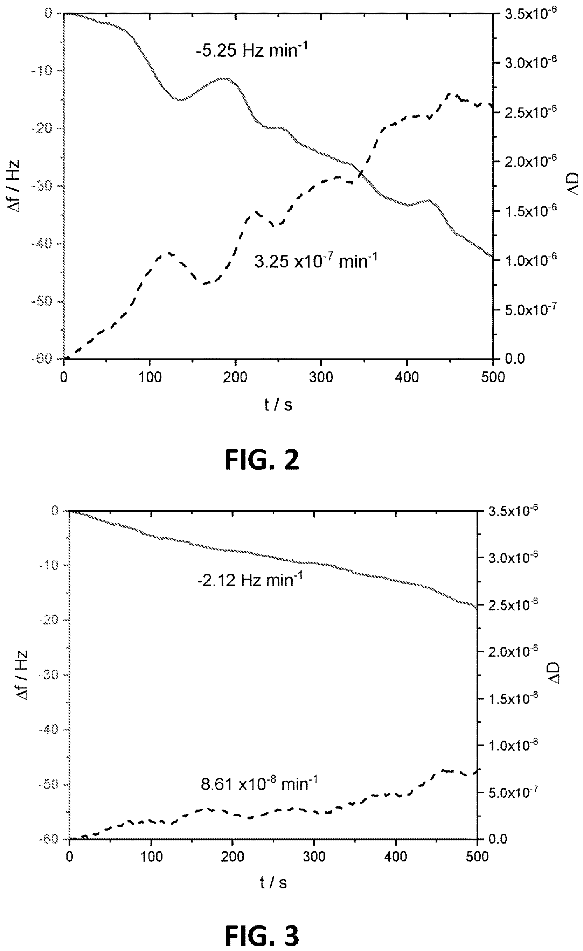

Figures 2 and 3 show the signal of the acoustic transducer after 8 minutes of sampling in a chamber in which it has been nebulised (Figure 2): PBS 1x; and (Figure 3): PBS 1x + [VLPs] = 10⁸ pfu/ml. The changes in the resonance frequency of the quartz crystal of the biosensor (8) are represented by a solid line, while the changes in the dissipation factor are represented by a broken line.

Lastly, a third object of the present invention is a virusometer for the detection of pathogens in air exhaled by a user, preferably to detect SARS-CoV-2, directly trapping the virus in the biosensor (8), as described in first object of the invention, comprising a layer of a specific antibody. The antibody is preferably immobilised by covalent anchoring, as described above.

The virusometer for the detection of pathogens in air exhaled by a user detects a pathogen, preferably SARS-CoV-2, by measuring the viral load directly in the exhaled air. It provides a signal related to the level of virus present in the air exhaled by a person and a real-time measurement.

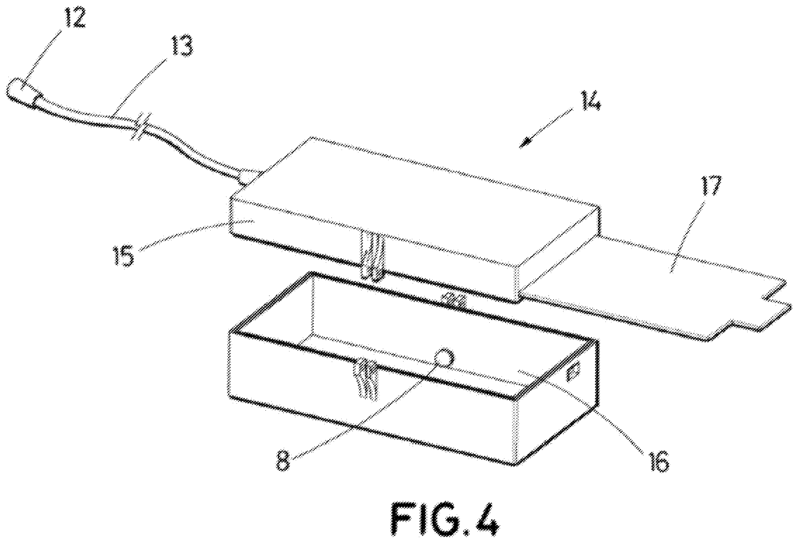

As shown in Figure 4, the virusometer for the detection of pathogens in air exhaled by a user comprises a mouthpiece (12) through which the user blows, in addition to a receptacle (14), divided by a separation piece of (17) movable in a first cavity (15) and a second cavity (16). The mouthpiece (12) is connected to the first cavity (15) by a 67 cm flexible plastic tube (13), in such a way that air fills this first cavity (15) when the user blows. This facilitates the homogenisation and preconcentration of the pathogen before the measurement.

Thus, when the blowing has finished, the separation element (17) moves, and thus the air reaches the second cavity (16), in which the biosensor (8) is arranged.

The biosensor (8), which can be connected to processing means to store and send data, identifies the pathogen load contained in the exhaled air, taking the measurement at an interval of three minutes.

As in the previously described biosensor (8), when the airborne pathogen or virion interacts with the antibodies immobilised on the biosensor chip (8), it causes a change in the resonance frequency and dissipation factor of the biosensor (8) located in the second cavity (16).

To increase the sensitivity thereof, the processing module does not directly determine the absolute dissipation values, but rather the rate of change of this parameter.

As indicated, the virusometer for the detection of pathogens in air exhaled by a user allows for the detection of other airborne viruses and microorganisms, mainly in aerosols, using natural or synthetic bioreceptors specific for desired pathogen.

## Claims

1. A virusometer (1) for the detection of pathogens in air, comprising:
- a sampling module, comprising:
- a first duct (3), with an air inlet (2) and an air outlet (10),
- an airflow measurement unit (4), positioned in the first duct (3),
- air mobilisation means (9), positioned in the first duct (3),
- a sample collection module, comprising:
- an aerosol collector (5), connected to the first duct (3),
- a virus detection module, comprising:
- a second duct (6), connected to the aerosol collector (5),
- fluid mobilisation means (7), arranged in the second duct (6), and
- and an acoustic transducer, which comprises a biosensor (8) for the detection of pathogens in air, which comprises a quartz crystal microbalance coated with one or more specific antibodies for at least one pathogen to be detected, anchored to the microbalance by an element selected among a generic antibody, a specific antibody, a protein, a hapten and a bioreceptor.

2. The virusometer (1) for the detection of pathogens in air of claim 1, wherein the antibodies of the biosensor (8) are specific anti-SARS-CoV-2.

3. The virusometer (1) for the detection of pathogens in air of claim 1, wherein the airflow measurement unit (4) is a flow meter.

4. The virusometer (1) for the detection of pathogens in air of claim 1, wherein the air mobilisation means (9) comprise a vacuum pump.

5. The virusometer (1) for the detection of pathogens in air of claim 1, wherein the fluid mobilisation means (7) comprise a peristaltic pump.

6. The virusometer (1) for the detection of pathogens in air of claim 1, which additionally comprises a funnel to prevent access of coarse particles, positioned at the inlet (2) of the first duct (3).

7. Virusometer for the detection of pathogens in air exhaled by a user, comprising:
- a receptacle (14),
- a separation piece (17), movable in the receptacle (14), such that it divides the same into a first cavity (15) and a second cavity (16),
- a mouthpiece (12), intended to receive the air exhaled by a user, connected to the first cavity (15) of the receptacle (14),
- a biosensor (8), for the detection of pathogens in air, which comprises a quartz crystal microbalance coated with one or more specific antibodies for at least one pathogen to be detected, anchored to the microbalance by an element selected among a generic antibody, a specific antibody, a protein, a hapten and a bioreceptor, the biosensor (8) being positioned in the second cavity (16) of the receptacle (14),
- processing means, connected to the biosensor (8).

8. The virusometer for the detection of pathogens in air exhaled by a user of claim 7, in which the antibodies of the biosensor (8) are specific anti-SARS-CoV-2.
